# EUROPÄISCHE PATENTSCHRIFT

(11) **EP 0 779 285 B1**
(45) Veröffentlichungstag und Bekanntmachung des Hinweises auf die Patenterteilung: **16.06.1999**
(21) Anmeldenummer: 96119399.2
(22) Anmeldetag: 04.12.1996
(51) Int. Cl.: C07D 307/62

(54) **Verfahren zur Herstellung von Ascorbinsäure**
Method for preparing ascorbid acid
Procédé de préparation de l'acide ascorbique

(30) Priorität: 16.12.1995 DE 19547073
(43) Veröffentlichungstag der Anmeldung: 18.06.1997
(73) Patentinhaber: MERCK PATENT GmbH, 64293 Darmstadt (DE)
(72) Erfinder: Fechtel, Ulrich, Dr., 64372 Ober-Ramstadt (DE); Wembacher, Karlheinz, 64319 Pfungstadt (DE)

(56) Entgegenhaltungen:
- DE-C- 19 547 073
- GB-A- 2 205 567
- J. FALBE, ED.: "Houben-Weyl, Methoden der organischen Chemie, Band E5/1" 1985 , THIEME VERLAG , STUTTGART DE XP002027080 * Seite 671 - Seite 672 *

## Beschreibung

Gegenstand der Erfindung ist ein Verfahren zur Herstellung von L-Ascorbinsäure durch Umsetzung von 2-Keto-L-Gulonsäure mit wässriger Mineralsäure in einem Lösungsmittelgemisch enthaltend ein inertes organisches Lösungsmittel und ein aliphatisches Keton, wobei man ein Säurechlorid hinzusetzt.

Gegenstand der Erfindung ist insbesondere ein Verfahren zur Herstellung von L-Ascorbinsäure, wobei man ein Gemisch bestehend aus 2-Keto-L-gulonsäure, einem inerten Lösungsmittel, einem Keton und einer wässrigen Mineralsäure erhitzt und danach mit einem Säurechlorid versetzt.

Bekannt sind Verfahren zur Herstellung von Ascorbinsäure in einem Schritt durch Umsetzung von 2-Keto-L-Gulonsäure mit einer Säure. In der US 2,185,383 ist die Umsetzung von 2-Keto-L-Gulonsäure mit konzentrierter Salzsäure und Essigsäure als Lösungsmittel beschrieben. In der japanischen ungeprüften Anmeldung 58-177986 ist ein Verfahren zur Herstellung von L-Ascorbinsäure beschrieben, bei dem zuerst eine Lösung von Natrium-2-Keto-L-Gulonsäure in Ethanol und Aceton mit Salzsäure neutralisiert wird. In der japanischen geprüften Anmeldung 48-15931 wird die Umsetzung von 2-Keto-L-Gulonsäure mit einer Mineralsäure in-einem inerten Lösungsmittel und in Gegenwart einer oberflächenaktiven Substanz beschrieben. Ebenso wird die Herstellung von L-Ascorbinsäure ausgehend von 2-Keto-L-Gulonsäureanhydrid in Gegenwart einer oberflächenaktiven Substanz in WO 87/00839 beschrieben. In EP 0 324 261 und in GB 2,205,567 wird die Umsetzung von 2-Keto-L-Gulonsäure mit einer Säure in einem Gemisch inerter Lösungsmittel in Gegenwart einer oberflächenaktiven Substanz beschrieben.

Überraschenderweise ergaben Untersuchungen im Rahmen der L-Ascorbinsäuresynthese, daß bei Zugabe von Säurechloriden auf oberflächenaktive Substanzen verzichtet werden kann. Das erfindungsgemäße neue Verfahren ermöglicht es, L-Ascorbinsäure durch Lactonisierung von 2-Keto-L-Gulonsäure mit wässriger Mineralsäure in einem Lösungsmittelgemisch unter Zusatz eines Säurechlorids in hohen Ausbeuten zu erhalten, die nicht niedriger liegen, als die in den schon bekannten Verfahren. Dies wird durch einen Vergleichsversuch aufgezeigt, der entsprechend den Angaben der Inhaltsstoffe in GB 2,205,567 durchgeführt wurde.

Mit dem Säurechlorid kann ein Teil des Wassers im System entfernt werden und somit Einfluß auf den Wassergehalt und damit auf die Ausbeute an Ascorbinsäure genommen werden.

Vorteilhaft erweist sich das erfindungsgemäße Verfahren auch in Bezug auf die einsetzbaren Mengen. So kann mit einer wesentlich höheren (2-4-fachen) Ketogulonsäuremenge pro Volumeneinheit gearbeitet werden und wobei Ausbeuten über 90 % d.Th. realisiert werden (Ausführungsbeispiel 2).

Das Lösungsmittelgemisch besteht aus einem inerten Lösungsmittel und einem aliphatischen Keton, wobei das Verhältnis Keton/inertes Lösungsmittel in einem weiten Bereich variiert werden kann.
Das Keton kann z.B. in gleicher Menge relativ zum inerten Lösungsmittel gegeben. Vorzugsweise gibt man 0,02-0,3 Vol.% Keton, besonders bevorzugt 0,03-0,1 Vol.% relativ zu 1 Vol.% des inerten Lösungsmittels hinzu.

Als inerte Lösungsmittel eignen sich z.B. Kohlenwasserstoffe wie Hexan, Heptan, Octan, Petrolether, Benzol, Toluol oder Xylol; chlorierte Kohlenwasserstoffe wie Chlorbenzol, Trichlorethylen, 1,2-Dichlorethan, Tetrachlorkohlenstoff, Chloroform oder Dichlormethan; Ether wie Tetrahydrofuran, Dioxan oder Isopropylether, gegebenenfalls auch Mischungen der Lösungsmittel untereinander.
Aliphatisches Keton bedeutet vorzugsweise Aceton, Methylethylketon, Diethylketon, Methylisopropylketon, ferner auch Cyclohexylketon.

Gegenstand der Erfindung ist auch ein Verfahren, dadurch gekennzeichnet, daß man 0,01 bis 1 Teil an Säurechlorid bezogen auf 1 Teil des inerten Lösungsmittels einsetzt Besonders bevorzugt ist ein Verhältnis von 0,1 bis 0,4 Teile an Säurechlorid bezogen auf 1 Teil des inerten Lösungsmittels.
Bevorzugt ist auch ein Verhältnis von 0,01 bis 0,2 Teile Säurechlorid bezogen auf 1 Teil 2-Keto-L-Gulonsäure.

Gegenstand der Erfindung ist auch ein Verfahren, wie oben beschrieben, bei dem das Säurechlorid ein aliphatisches Carbonsäurechlorid mit 1 bis 4 C-Atomen, insbesondere Acetylchlorid, Propionylchlorid, Phosgen, Diphosgen, Oxalylchlorid, ferner auch Malonsäuredichlorid, Bernsteinsäuredichlorid oder Butyrylchlorid bedeutet.

Säurechlorid bedeutet vorzugsweise auch Thionylchlorid, ferner Phosphoroxichlorid.

Die Umsetzung von 2-Keto-L-Gulonsäure wird in einem, wie oben beschrieben, Lösungsmittelgemisch durchgeführt. Die Konzentration der 2-Keto-L-Gulonsäure ist in bezug auf das Lösungsmittelgemisch nicht beschränkt. Bevorzugt sind 5-40 Gewichtsprozent 2-Keto-L-Gulonsäure.

Wässrige Mineralsäure bedeutet z.B. Salzsäure, Phosphorsäure oder Schwefelsäure. Bevorzugt werden 0,2 bis 2 Mol Mineralsäure bezogen auf 1 Mol 2-Keto-L-Gulonsäure eingesetzt.

Gegenstand der Erfindung ist auch ein Verfahren, wie beschrieben, dadurch gekennzeichnet, daß man die Reaktion bei Temperaturen zwischen 40 und 100 °C durchführt. Bevorzugt ist der Temperaturbereich zwischen 50 und 80 °C, ganz besonders bevorzugt ist der Temperaturbereich zwischen 55 und 70° C.

Die Aufarbeitung erfolgt nach an sich bekannten Methoden, z.B. durch Filtration, Entfernen des Lösungsmittels, Extraktion und/ oder Kristallisation.
Entsprechend dem erfindungsgemäßen Verfahren liegen die Ausbeuten an L-Ascorbinsäure in der Regel über 90 %, so daß aufwendige Reinigungsschritte entfallen können.
Vor- und nachstehend sind alle Temperaturen in °C angegeben. Die Gehaltsbestimmungen (Reinheit) erfolgten z.B. durch iodometrische Bestimmung des Ascorbinsäuregehaltes im massemäßig erfaßten Rohprodukt.

### Beispiel 1

Eine Lösung aus 100 g 2-Keto-L-Gulonsäure (97,7 %, 2,3 % Wasser) in 575 ml Toluol wird mit 30 ml Aceton und 18,3 ml 37 %iger Salzsäure versetzt und 1 Stunde auf 60° erhitzt. Anschließend wird ein Gemisch bestehend aus 9,4 ml Acetylchlorid und 50 ml Aceton zugesetzt und die Mischung 7 Stunden bei 60° gehalten. Die Salzsäure wird durch Destillation entfernt. Der angefallene Feststoff wird abfiltriert, der Filterkuchen mit 50 ml Toluol nachgewaschen und getrocknet. Man erhält 85,9 g L-Ascorbinsäure; Reinheit: 94,6 %; Ausbeute: 91,7 % d.Th.

### Beispiel 2

Eine Lösung aus 200 g 2-Keto-L-Gulonsäure (97,7 %, 2,3 % Wasser) in 170 ml Toluol, 80 ml Aceton und 20 ml 37 % iger HCl wird 2 Stunden auf 60° erhitzt. Anschließend wird ein Gemisch bestehend aus 18,8 ml Acetylchlorid in 40 ml Aceton und 16,6 ml 37 % iger HCl in 40 Aceton zugesetzt und die Mischung 7 Stunden bei 60° gehalten. Die Aufarbeitung erfolgt analog Beispiel 1.
Man erhält 170,4 g Ascorbinsäure; Reinheit 94,4 %: Ausbeute: 91,4 % d.Th.

### Beispiel 3

Eine Lösung aus 100 g 2-Keto-L-Gulonsäure (97,7 %, 2,3 % Wasser) in 570 ml Toluol, 30 ml Aceton und 10 ml 37 % iger HCI wird 1 Stunden auf 60° erhitzt. Anschließend wird ein Gemisch bestehend aus 8,8 g Thionylchlorid in 50 ml Aceton und 6,7 ml 37 % iger HCI zugesetzt und die Mischung 4 Stunden bei 60° gehalten. Die Aufarbeitung erfolgt analog Beispiel 1.
Man erhält 86,3 g Ascorbinsäure; Reinheit 94,4 %; Ausbeute: 91,9 % d.Th.

### Beispiel 4

Eine Lösung aus 100 g 2-Keto-L-Gulonsäure (97,7 %, 2,3 % Wasser) in 575 ml Toluol, 30 ml Aceton und 10 ml 37 % iger HCI wird 3 Stunden auf 60° erhitzt. Anschließend wird ein Gemisch bestehend aus 7,3 g Diphosgen in 50 ml Aceton und 7,0 ml 37 % iger HCI zugesetzt und die Mischung 8 Stunden bei 60° gehalten. Die Aufarbeitung erfolgt analog Beispiel 1.
Man erhält 85,0 g Ascorbinsäure; Reinheit 95,4 %; Ausbeute: 91,5 % d.Th.

### Vergleichsversuch

Eine Lösung aus 100 g 2-Keto-L-Gulonsäure (97,7 %, 2,3 % Wasser) in 575 ml Toluol, 30 ml Aceton und 10 ml 37 % iger HCI wird 3 Stunden auf 60° erhitzt. Anschließend wird ein Gemisch bestehend 5,4 g dest. Wasser 10 ml 37 % ige Salzsäure, 0,117 g N-Cetyl-N,N,N-trimethylammoniumbromid und 52,2 g HCI/Aceton (22,2 % ig) zugesetzt und die Mischung 8 Stunden bei 60° gehalten. Die Aufarbeitung erfolgt analog Beispiel 1.
Man erhält 85,4 g Ascorbinsäure; Reinheit 93,5 %; Ausbeute: 90,1 % d.Th.

## Patentansprüche

1. Verfahren zur Herstellung von L-Ascorbinsäure durch Umsetzung von 2-Keto-L-Gulonsäure mit wässriger Mineralsäure in einem Lösungsmittelgemisch enthaltend ein inertes organisches Lösungsmittel und ein aliphatisches Keton, dadurch gekennzeichnet, daß man ein Säurechlorid hinzusetzt.

2. Verfahren nach Anspruch 1, dadurch gekennzeichnet, daß man 0,01 bis 1 Teil an Säurechlorid bezogen auf 1 Teil des inerten Lösungsmittels einsetzt.

3. Verfahren nach Anspruch 1 oder 2, dadurch gekennzeichnet, daß man die Reaktion bei Temperaturen zwischen 40 und 100 °C durchführt.

4. Verfahren nach einem der vorangehenden Ansprüche, dadurch gekennzeichnet, daß man ein Gemisch bestehend aus 2-Keto-L-gulonsäure, einem inerten Lösungsmittel, einem Keton und einer wässrigen Mineralsäure erhitzt und danach mit einem Säurechlorid versetzt.

5. Verfahren nach einem der vorangehenden Ansprüche, dadurch gekennzeichnet, daß das Säurechlorid ein aliphatisches Carbonsäurechlorid mit 1 bis 4 C-Atomen ist.

6. Verfahren nach einem der vorangehenden Ansprüche, dadurch gekennzeichnet, daß das Säurechlorid Thionylchlorid ist.

7. Verfahren nach Anspruch 5, dadurch gekennzeichnet, daß das Säurechlorid ein aliphatisches Carbonsäurechlorid ausgewählt aus der Gruppe bestehend aus Acetylchlorid, Oxalylchlorid, Phosgen und Diphosgen ist.

## Claims

1. Process for the preparation of L-ascorbic acid by reacting 2-keto-L-gulonic acid with aqueous mineral acid in a solvent mixture containing an inert organic solvent and an aliphatic ketone, characterized in that an acid chloride is added.

2. Process according to Claim I, characterized in that 0.01 to 1 part of acid chloride relative to 1 part of the inert solvent is employed.

3. Process according to Claim 1 or 2, characterized in that the reaction is carried out at temperatures between 40 and 100°C.

4. Process according to one of the preceding claims, characterized in that a mixture consisting of 2-keto-L-gulonic acid, an inert solvent, a ketone and an aqueous mineral acid is heated and subsequently an acid chloride is added.

5. Process according to one of the preceding claims, characterized in that the acid chloride is an aliphatic carboxylic acid chloride having 1 to 4 C atoms.

6. Process according to one of the preceding claims, characterized in that the acid chloride is thionyl chloride.

7. Process according to Claim 5, characterized in that the acid chloride is an aliphatic carboxylic acid chloride selected from the group comprising acetyl chloride, oxalyl chloride, phosgene and diphosgene.

## Revendications

1. Procédé pour la préparation de l'acide L-ascorbique par la réaction de l'acide 2-céto-L-gulonique avec un acide minéral aqueux, dans un mélange de solvants contenant un solvant organique inerte et une cétone aliphatique, caractérisé en ce que l'on ajoute un chlorure d'acide.

2. Procédé selon la revendication 1, caractérisé en ce que l'on utilise 0,01 à 1 partie de chlorure d'acide par rapport à 1 partie du solvant inerte.

3. Procédé selon la revendication 1 ou 2, caractérisé en ce que l'on effectue la réaction à des températures comprises entre 40 et 100°C.

4. Procédé selon l'une des revendications précédentes, caractérisé en ce que l'on chauffe un mélange constitué d'acide 2-céto-L-gulonique, d'un solvant inerte, d'une cétone et d'un acide minéral aqueux, et on y ajoute ensuite un chlorure d'acide.

5. Procédé selon l'une des revendications précédentes, caractérisé en ce que le chlorure d'acide est un chlorure d'acide carboxylique aliphatique ayant de 1 à 4 atomes de carbone.

6. Procédé selon l'une des revendications précédentes, caractérisé en ce que le chlorure d'acide est le chlorure de thionyle.

7. Procédé selon la revendication 5, caractérisé en ce que le chlorure d'acide est un chlorure d'acide carboxylique aliphatique choisi dans le groupe constitué par le chlorure d'acétyle, le chlorure d'oxalyle, le phosgène et le diphosgène.
